# EUROPEAN PATENT SPECIFICATION

(11) **EP 4 662 873 B1**
(45) Date of publication and mention of the grant of the patent: **13.05.2026**
(21) Application number: 24720635.2
(22) Date of filing: 14.03.2024
(51) Int. Cl.: H04R 1/10, A61B 5/08, A61B 5/00

(54) **USER-FOCUSED COUGHING DETECTION USING ACTIVE ACOUSTIC SENSING**
BENUTZERFOKUSSIERTE HUSTENERKENNUNG MIT AKTIVER AKUSTISCHER ERFASSUNG
DÉTECTION DE TOUX AXÉE SUR L'UTILISATEUR À L'AIDE D'UNE DÉTECTION ACOUSTIQUE ACTIVE

(30) Priority: 16.03.2023 US 202363490657 P
(43) Date of publication of application: 17.12.2025
(73) Proprietor: GOOGLE LLC, Mountain View CA 94043 (US)
(72) Inventor: FAN, Xiaoran, Mountain View, CA 94043 (US); THORMUNDSSON, Trausti, Mountain View, CA 94043 (US)
(74) Representative: Maikowski & Ninnemann Patentanwälte Partnerschaft mbB
(86) International application number: PCT/US2024/019953
(87) International publication number: WO 2024/192250

(56) References cited:
- US-A1- 2014 051 940
- US-A1- 2022 087 570
- US-A1- 2022 386 959
- RÖDDIGER TOBIAS ET AL: "Sensing with Earables", PROCEEDINGS OF THE ACM ON INTERACTIVE, MOBILE, WEARABLE AND UBIQUITOUS TECHNOLOGIES, ACMPUB27, NEW YORK, NY, USA, vol. 6, no. 3, 7 September 2022 (2022-09-07), pages 1 - 57, XP058925371, DOI: 10.1145/3550314

## Description

### BACKGROUND

Wireless technology has become prevalent in everyday life, making communication and data readily accessible to users. One type of wireless technology are wireless hearables, examples of which include wireless earbuds and wireless headphones. Wireless hearables have allowed users freedom of movement while listening to audio content from music, audio books, podcasts, and videos. With the prevalence of wireless hearables, there is a market for adding additional features to existing hearables without introducing hardware changes. US 2022 / 0 386 959 A1 and US 2022 / 0 087 570 A1 disclose methods for determining that a user coughed during a time period. US 2014 / 0 051 940A1 discloses a method for determining blood pulse, based on a transmitted and received acoustic signal that propagates with an ear canal of a user. Sensing methods using ear-mounted devices are disclosed in RÖDDIGER TOBIAS ET AL: "Sensing with Earables", PROCEEDINGS OF THE ACM ON INTERACTIVE, MOBILE, WEARABLE AND UBIQUITOUS TECHNOLOGIES, ACMPUB27, NEW YORK, NY, USA, vol. 6, no. 3, 7 September 2022 (2022-09-07), pages 1-57, XP058925371.

### SUMMARY

A claimed solution is specified by a method according to claim 1, by a non-transitory computer-readable storage medium according to claim 10 and by a system according to claim 11. Dependent claims specify embodiments thereof. Techniques and apparatuses are described for performing user-focused coughing detection using active acoustic sensing. During active acoustic sensing, a hearable transmits and receives at least one acoustic signal, which propagates within a user's ear canal. This acoustic signal can be modulated by muscle movements caused by the user coughing. As the acoustic signal propagates within the user's ear canal, it remains substantially isolated from external environmental noise. As such, active acoustic sensing can perform user-focused coughing detection. In contrast to other coughing detection techniques, user-focused coughing detection enables monitoring of the user's coughing while substantially preventing the generation of false positives caused by an external source (e.g., another person or media) that is in proximity to the user and/or the hearable. Accordingly, active acoustic sensing can provide accurate and reliable coughing detection that can discriminate between the user and another outside source.

User-focused coughing detection can be useful for health monitoring and enabling early detection of an illness. In addition to being relatively unobtrusive, some hearables can be configured to perform user-focused coughing detection using active acoustic sensing without the need for additional hardware. As such, the size, cost, and power usage of the hearable can help make health monitoring accessible to a larger group of people and improve the user experience with hearables.

Aspects described below include a method for performing user-focused coughing detection using active acoustic sensing. The method includes transmitting, with a hearable, during a first time period, an acoustic transmit signal that propagates within at least a portion of an ear canal of a user. The method also includes receiving, with the hearable, during the first time period, an acoustic receive signal The acoustic receive signal represents a version of the acoustic transmit signal with one or more characteristics modified based on the propagation within the ear canal and based on the user coughing during at least a portion of the first time period. The method additionally includes determining that the user coughed during the first time period based on changes in an amplitude and/or phase of the acoustic receive signal due to the deformation of the ear canal.

The method can optionally include generating a control signal that controls an operation of a device based on the determination that the user coughed. The device may comprise at least one of the hearable or a computing device that is coupled to the hearable.

Aspects described below include a computer-readable storage medium comprising instructions that, responsive to execution by a processor, cause a hearable to perform any one of the methods described herein. In one example, the at least one processor may be part of the device of which an operation is controlled based on the detected coughing or the at least one processor may be part of a hearable coupled to the device of which an operation is controlled based on the detected coughing.

Aspects described below include a system comprising a hearable with at least one transducer and comprising at least one processor. The system is configured to perform, using the at least one transducer and the at least one processor, any one of the methods described herein. For example, the at least one transducer may be configured to transmit the acoustic transmit signal and/or to receive the acoustic receive signal.

Aspects described below include a system with means for performing user-focused coughing detection using active acoustic sensing.

### BRIEF DESCRIPTION OF DRAWINGS

Apparatuses for and techniques that perform user-focused coughing detection using active acoustic sensing are described with reference to the following drawings. The same numbers are used throughout the drawings to reference like features and components:
FIG. 1 illustrates an example environment in which active acoustic sensing can be implemented;
FIG. 2 illustrates example environments for performing user-focused coughing detection using active acoustic sensing;
FIG. 3 illustrates example components of a computing device;
FIG. 4 illustrates example components of a hearable;
FIG. 5 illustrates example operations of two hearables;
FIG. 6 illustrates an example implementation of a hearable capable of performing user-focused coughing detection using active acoustic sensing;
FIG. 7 illustrates example pre-processed signals for performing aspects of user-focused coughing detection using active acoustic sensing;
FIG. 8 illustrates other example pre-processed signals for performing aspects of user-focused coughing detection using active acoustic sensing;
FIG. 9 illustrates a first example method for performing aspects of user-focused coughing detection using active acoustic sensing;
FIG. 10 illustrates a second example method for performing aspects of user-focused coughing detection using active acoustic sensing;
FIG. 11 illustrates a third example method for performing aspects of user-focused coughing detection using active acoustic sensing; and
FIG. 12 illustrates an example computing system embodying, or in which techniques may be implemented that enable use of, user-focused coughing detection using active acoustic sensing.

### DETAILED DESCRIPTION

Currently, when a person is potentially exposed to a contagious disease, such as a coronavirus or Ebola, the person can be placed in quarantine, instructed to regularly visit a hospital or doctor's office, or asked to track their own health. Each of these methods, however, has significant drawbacks. Placing a person in quarantine is expensive, can be illegal absent some imminent threat, and removes the quarantined persons from his or her productive pursuits. Asking a person to regularly visit a facility to check their health has highly irregular results and, due to the artificial atmosphere in which health monitoring is performed, is often not reliable at catching an infection early. Asking a person to track their own health also has many drawbacks, including compliance, poor data, and failure to detect the infection before others are infected and often after the best outcome for that person can be attained through early detection.

To enable a person to track their own health, some devices support health monitoring. These devices, however, can be costly, obtrusive, uncomfortable to wear, and/or cumbersome to set up. As such, people may choose to forego health monitoring if the device is too expensive, negatively impacts their movement, or causes inconveniences while performing daily activities. It is therefore desirable for health-monitoring devices to be reliable, portable, and affordable to encourage more users to take advantage of these features.

Coughing is a symptom associated with many illnesses. Some devices can detect coughing by monitoring over-the-air voice signals using a microphone or a voice accelerometer. These devices, however, are susceptible to noise that is present in an external environment. This susceptibility can make it challenging for a device to distinguish between coughing that originates from the user or coughing that originates from another source. A first example source can include another person that coughs proximate to the user and/or the device. A second example source can be media that plays a coughing sound. Without an ability to identify coughing as coming from the user, these devices may generate inaccurate information regarding the health of the user.

To improve aesthetics and reduce encumbrance, it can be desirable to design hearables with smaller sizes. As space becomes limited, it can be challenging to integrate additional components, such as the voice accelerometer, within the hearables. With the prevalence of hearables, there is a market for adding additional features to existing hearables to facilitate health monitoring without introducing hardware changes.

Provided according to one or more preferred embodiments is a hearable, such as an earbud, that is capable of performing a novel physiological monitoring process termed herein audioplethysmography. Audioplethysmography is an active acoustic method capable of sensing subtle physiologically-related changes observable at a user's outer and middle ear. Instead of relying on other auxiliary sensors, such as optical or electrical sensors, audioplethysmography involves transmitting and receiving acoustic signals that at least partially propagate within a user's ear canal. To perform audioplethysmography, the hearable forms at least a partial seal in or around the user's outer ear. This seal enables formation of an acoustic circuit, which includes the seal, the hearable, the ear canal, and an ear drum of the ear. By transmitting and receiving acoustic signals, the hearable can recognize changes in the acoustic circuit caused by the user coughing. In this manner, active acoustic sensing enables the hearable to detect instances in which the user coughs.

Active acoustic sensing can detect a variety of different types of coughs, including a dry cough, a wet cough, a croup cough, or a paroxysmal cough. In some implementations, active acoustic sensing can be used to distinguish between these different types of coughs, which provides additional information for health monitoring, disease diagnosis, and treatment. The techniques for performing user-focused coughing detection can also be adapted and applied to detecting other similar actions, such as choking and/or throat clearing. Active acoustic sensing can also be used to provide other biometric information to assist with early illness detection. Example biometric information can include the user's heart rate, heart rate variability, respiration rate, and/or blood pressure.

During active acoustic sensing, a hearable transmits and receives at least one acoustic signal, which propagates within the user's ear canal. This acoustic signal can be modulated by the user coughing. More specifically, the modulation is caused by the muscle movements associated with coughing and/or the sound produced by the coughing. As the acoustic signal propagates within the user's ear canal, it is substantially isolated from external environmental noise. As such, active acoustic sensing can perform user-focused coughing detection. In contrast to other coughing detection techniques, user-focused coughing detection enables monitoring of the user's coughing while substantially preventing the generation of false positives caused by an external source (e.g., another person or media) that is in proximity to the user and/or the hearable. Accordingly, active acoustic sensing can provide accurate and reliable coughing detection that can discriminate between the user and another outside source.

User-focused coughing detection can be useful for health monitoring and enabling early detection of an illness. In addition to being relatively unobtrusive, some hearables can be configured to perform user-focused coughing detection using active acoustic sensing without the need for additional hardware. As such, the size, cost, and power usage of the hearable can help make health monitoring processing accessible to a larger group of people and improve the user experience with hearables. The user has the ability to enable and/or disable user-focused coughing detection. Also, the data associated with user-focused coughing detection can be made private. In general, individual users may have constant control over what programs can or cannot do with the data that is collected based on user-focused coughing detection.

### Operating Environment

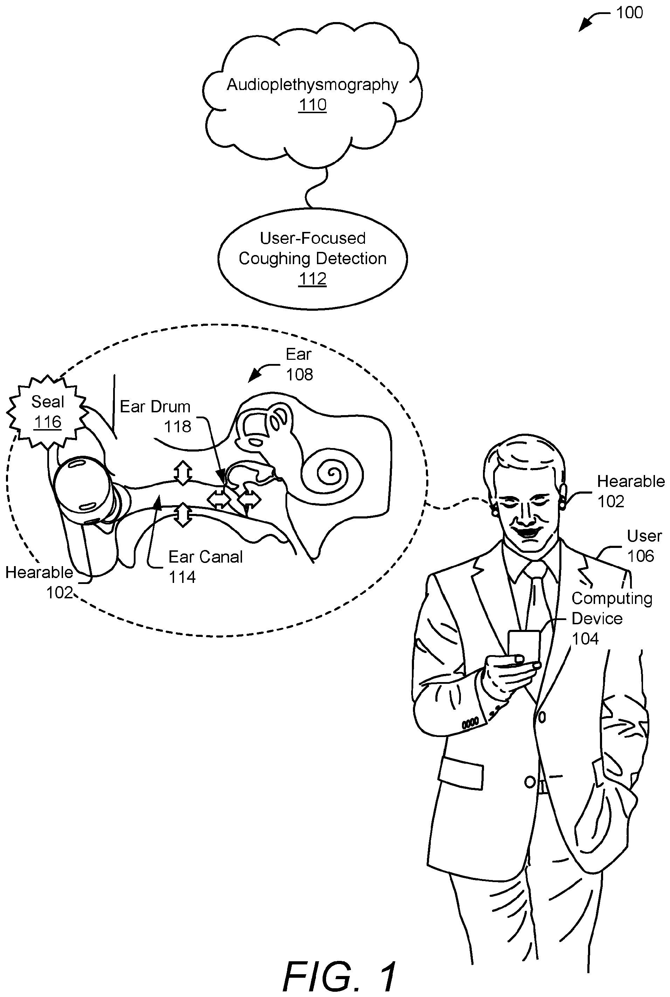

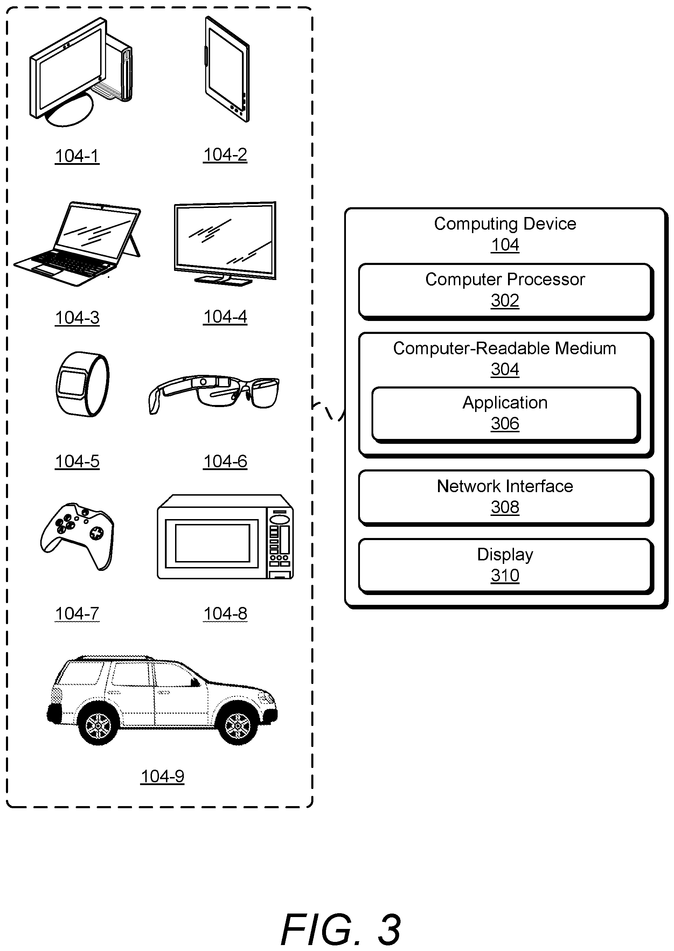

FIG. 1 is an illustration of an example environment 100 in which active acoustic sensing can be implemented. In the example environment 100, a hearable 102 is connected to a computing device 104 using a physical or wireless interface. The hearable 102 is a device that can play audible content provided by the computing device 104 and direct the audible content into a user 106's ear 108. In this example, the hearable 102 operates together with the computing device 104. In other examples, the hearable 102 can operate or be implemented as a stand-alone device. Although depicted as a smartphone, the computing device 104 can include other types of devices, including those described with respect to FIG. 3.

The hearable 102 is capable of performing audioplethysmography 110, which is an active acoustic method of sensing that occurs at the ear 108. The hearable 102 can perform this sensing without the use of other auxiliary sensors, such as an optical sensor or an electrical sensor. Through audioplethysmography 110, the hearable 102 can perform user-focused coughing detection 112. Additionally, the hearable 102 can also use audioplethysmography 110 to perform biometric monitoring. With user-focused coughing detection 112, the hearable 102 can accurately and reliably recognize when the user 106 coughs while minimizing false positives caused by environmental noise (e.g., another person coughing or media playing a coughing sound).

A variety of different types of coughs can be detected using user-focused coughing detection 112, including a dry cough, a wet cough, a croup cough, or a paroxysmal cough. Aspects of user-focused coughing detection 112 can also be adapted to encompass choking detection and/or throat clearing detection. The hearable 102 can also use audioplethysmography 110 to distinguish between coughing and other types of actions, such as those associated with chewing, talking, or other physiological metrics.

To perform user-focused coughing detection 112, the hearable 102 uses audioplethysmography 110 to detect subtle pressure waves that propagate to the user 106's ear canal 114. These pressure waves modify characteristics of acoustic signals that are transmitted and received by the hearable 102 and propagate through the ear canal 114. As the user 106 coughs, the ear canal 114 deforms at least in part due to the muscle movements made by the user 106 as they cough. Additionally or alternatively, the ear canal 114 can deform due to the sound created by the cough, which can travel to the ear canal 114 via bone conduction.

To use audioplethysmography 110, the user 106 positions the hearable 102 in a manner that creates at least a partial seal 116 around or in the ear 108. Some parts of the ear 108 are shown in FIG. 1, including the ear canal 114 and an ear drum 118 (or tympanic membrane). Due to the seal 116, the hearable 102, the ear canal 114, and the ear drum 118 couple together to form an acoustic circuit. Audioplethysmography 110 involves, at least in part, measuring properties associated with this acoustic circuit. The properties of the acoustic circuit can change due to a variety of different situations or actions.

For example, consider a change that occurs in a physical structure of the ear 108. Example changes to the physical structure include a change in a geometric shape of the ear canal 114 and/or a change in a volume of the ear canal 114. This change can be caused, at least in part, by a pressure wave that is formed by the user 106 coughing. For instance, the tissue around the ear canal 114 and the ear drum 118 itself are slightly "squeezed" due to the pressure wave. This squeeze causes a volume of the ear canal 114 to be slightly reduced. As the squeezing subsides, the volume of the ear canal 114 is slightly increased. The increasing and decreasing of the volume of the ear canal 114 is indicated by the arrows in FIG. 1. The physical changes within the ear 108 can modulate an amplitude, frequency, and/or phase of an acoustic signal that propagates through the ear canal 114.

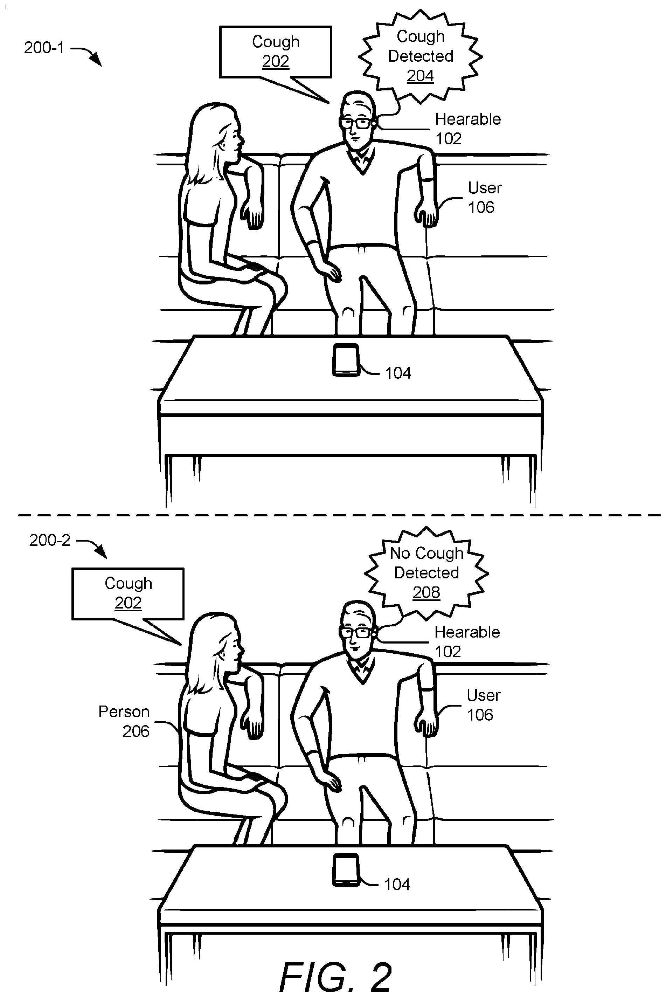

The techniques for audioplethysmography 110 can be performed while the hearable 102 is rendering (e.g., playing or transmitting) audible content and/or while the user 106 is actively moving or performing an activity (e.g., walking or running). As such, active acoustic sensing enables the hearable 102 to perform user-focused coughing detection 112 in a variety of different situations. One such situation is further described with respect to FIG. 2.

FIG. 2 illustrates example environments 200-1 and 200-2 for performing aspects of user-focused coughing detection 112 using active acoustic sensing. In the environment 200-1, the user 106 coughs 202 while wearing at least one hearable 102. With audioplethysmography 110, the hearable 102 detects that the user 106 is coughing, as indicated at 204. The hearable 102 can also communicate this information to the computing device 104. With this information, the computing device 104 can keep track of how often the user 106 coughs and provide this information to the user 106 or a doctor specified by the user 106.

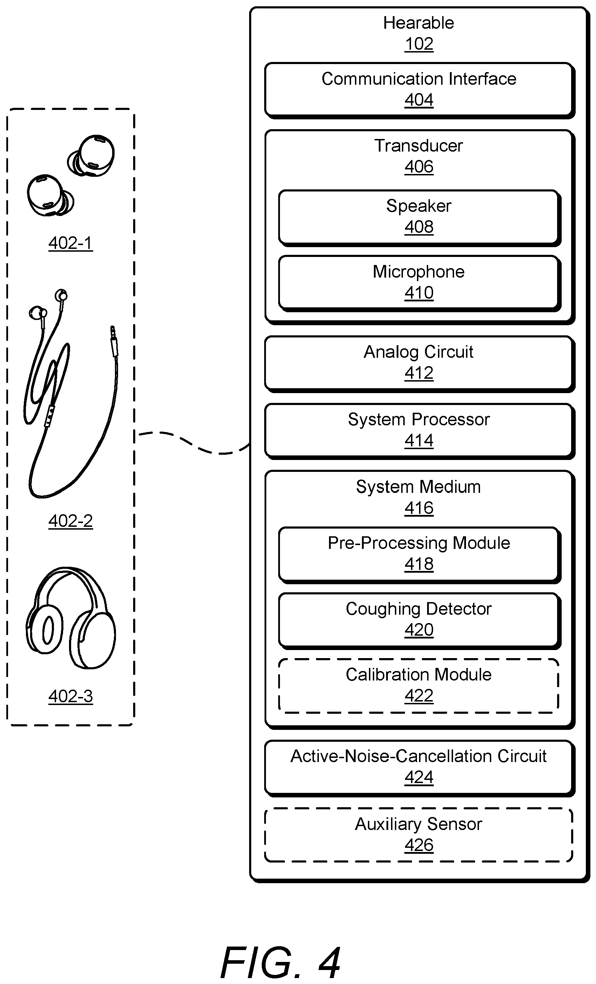

In the environment 200-2, a person 206 that is proximate to the hearable 102 coughs 202. The person 206 is not wearing the hearable 102, and is therefore not considered a user of the hearable 102. With audioplethysmography 110, the hearable 102 determines that the user 106 did not cough, as shown at 208. With the ability to detect the user 106 coughing without detecting the person 206 coughing, the hearable 102 can provide reliable user-focused coughing detection 112 and assist with identifying a possible infection quickly compared to other coughing-detection techniques. The computing device 104 and the hearable 102 are further described with respect to FIGs. 3 and 4, respectively.

FIG. 3 illustrates an example implementation of the computing device 104. The computing device 104 is illustrated with various non-limiting example devices including a desktop computer 104-1, a tablet 104-2, a laptop 104-3, a television 104-4, a computing watch 104-5, computing glasses 104-6, a gaming system 104-7, a microwave 104-8, and a vehicle 104-9. Other devices may also be used, such as an augmented and/or virtual reality headset, a home service device, a smart speaker, a smart thermostat, a baby monitor, a Wi-Fi^{™} router, a drone, a trackpad, a drawing pad, a netbook, an e-reader, a home automation and control system, a wall display, and another home appliance. Note that the computing device 104 can be wearable, non-wearable but mobile, or relatively immobile (e.g., desktops and appliances).

The computing device 104 includes one or more computer processors 302 and at least one computer-readable medium 304, which includes memory media and storage media. Applications and/or an operating system (not shown) embodied as computer-readable instructions on the computer-readable medium 304 can be executed by the computer processor 302 to provide some of the functionalities described herein. The computer-readable medium 304 can optionally include an application 306, which can utilize some aspect of user-focused coughing detection 112. Generally speaking, the application 306 can use information provided by the hearable 102 to perform an action. Example actions can include monitoring the user 106's coughing and/or health based on the user-focused coughing detection 112. More specifically, the application 306 can keep track of the time of day that the user 106 coughs, how often the user 106 coughs, the type of cough, and so forth. With this information, the application 306 can inform the user 106 if their coughing may indicate a potential illness. Optionally, the application 306 can also use biometric information provided by the hearable 102 for health monitoring and/or for enhancing the early detection of an illness.

The computing device 104 can also include a network interface 308 for communicating data over wired, wireless, or optical networks. For example, the network interface 308 may communicate data over a local-area-network (LAN), a wireless local-area-network (WLAN), a personal-area-network (PAN), a wire-area-network (WAN), an intranet, the Internet, a peer-to-peer network, point-to-point network, a mesh network, Bluetooth^{®}, and the like. The computing device 104 may also include the display 310. Although not explicitly shown, the hearable 102 can be integrated within the computing device 104, or can connect physically or wirelessly to the computing device 104. The hearable 102 is further described with respect to FIG. 4.

FIG. 4 illustrates an example hearable 102. The hearable 102 is illustrated with various non-limiting example devices, including wireless earbuds 402-1, wired earbuds 402-2, and headphones 402-3. Other example hearables 102 can also include hearing aids. The earbuds 402-1 and 402-2 are a type of in-ear device that fits into the ear canal 114. Each earbud 402-1 or 402-2 can represent a hearable 102. Headphones 402-3 can rest on top of or over the ears 108. The headphones 402-3 can represent closed-back headphones, open-back headphones, on-ear headphones, or over-ear headphones. Each headphone 402-2 includes two hearables 102, which are physically packaged together. In general, there is one hearable 102 for each ear 108. The headphones 402-3 may be designed in some manner or may utilize techniques, such as beamforming, to assist with directing signals used for audioplethysmography 110 into the ear canal 114.

The hearable 102 includes a communication interface 404 to communicate with the computing device 104, though this need not be used when the hearable 102 is integrated within the computing device 104. The communication interface 404 can be a wired interface or a wireless interface, in which audio content is passed from the computing device 104 to the hearable 102. The hearable 102 can also use the communication interface 404 to pass data associated with audioplethysmography 110, user-focused coughing detection 112, and/or biometric monitoring to the computing device 104. In general, the data provided by the communication interface 404 is in a format usable by the application 306 or the computing device 104.

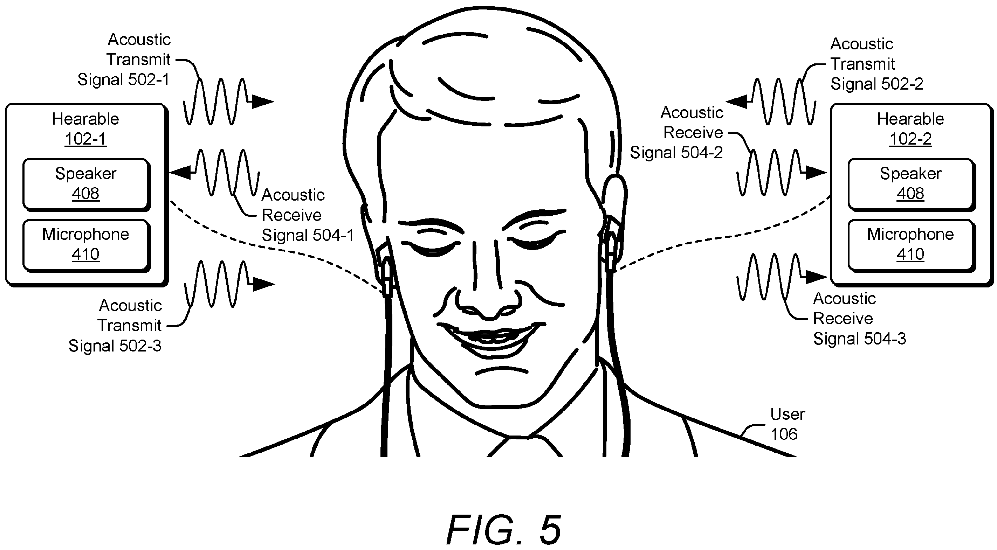

The communication interface 404 also enables the hearable 102 to communicate with another hearable 102. During bistatic sensing, for instance, the hearable 102 can use the communication interface 404 to coordinate with the other hearable 102 to support two-ear audioplethysmography 110, as further described with respect to FIG. 5. In particular, the transmitting hearable 102 can communicate timing and waveform information to the receiving hearable 102 to enable the receiving hearable 102 to appropriately demodulate a received acoustic signal.

The hearable 102 includes at least one transducer 406 that can convert electrical signals into sound waves. The transducer 406 can also detect and convert sound waves into electrical signals. These sound waves may include ultrasonic frequencies, which may be used for audioplethysmography 110. In particular, a frequency spectrum (e.g., range of frequencies) that the transducer 406 uses to generate an acoustic signal can include frequencies from a low-end of the audible range to a high-end of the ultrasonic range, e.g., between 20 hertz (Hz) to 2 megahertz (MHz). Other example frequency spectrums for audioplethysmography 110 can encompass frequencies between 20 Hz and 20 kilohertz (kHz), between 20 kHz and 2 MHz, between 20 and 60 kHz, or between 30 and 40 kHz.

In an example implementation, the transducer 406 has a monostatic topology. With this topology, the transducer 406 can convert the electrical signals into sound waves and convert sound waves into electrical signals (e.g., can transmit or receive acoustic signals). Example monostatic transducers may include piezoelectric transducers, capacitive transducers, and micro-machined ultrasonic transducers (MUTs) that use microelectromechanical systems (MEMS) technology.

Alternatively, the transducer 406 can be implemented with a bistatic topology, which includes multiple transducers that are physically separate. In this case, a first transducer converts the electrical signal into sound waves (e.g., transmits acoustic signals), and a second transducer converts sound waves into an electrical signal (e.g., receives the acoustic signals). An example bistatic topology can be implemented using at least one speaker 408 and at least one microphone 410. The speaker 408 and the microphone 410 can be dedicated for audioplethysmography 110 or can be used for both audioplethysmography 110 and other functions of the computing device 104 (e.g., passive audio sensing, presenting audible content to the user 106, capturing the user 106's voice for a phone call, or for voice control).

In general, the speaker 408 and the microphone 410 are directed towards the ear canal 114 (e.g., oriented towards the ear canal 114). Accordingly, the speaker 408 can direct acoustic signals towards the ear canal 114, and the microphone 410 is responsive to receiving acoustic signals from the direction associated with the ear canal 114.

The hearable 102 includes at least one analog circuit 412, which includes circuitry and logic for conditioning electrical signals in an analog domain. The analog circuit 412 can include analog-to-digital converters, digital-to-analog converters, amplifiers, filters, mixers, and switches for generating and modifying electrical signals. In some implementations, the analog circuit 412 includes other hardware circuitry associated with the speaker 408 or microphone 410.

The hearable 102 also includes at least one system processor 414 and at least one system medium 416 (e.g., one or more computer-readable storage media). In the depicted configuration, the system medium 416 includes a pre-processing module 418 and a coughing detector 420. The system medium 416 also optionally includes a calibration module 422. The pre-processing module 418, the coughing detector 420, and the calibration module 422 can be implemented using hardware, software, firmware, or a combination thereof. In this example, the system processor 414 implements the pre-processing module 418, the coughing detector 420, and the calibration module 422. In an alternative example, the computer processor 302 of the computing device 104 can implement at least a portion of the pre-processing module 418, the coughing detector 420, and/or the calibration module 422. In this case, the hearable 102 can communicate digital samples of the acoustic signals to the computing device 104 using the communication interface 404.

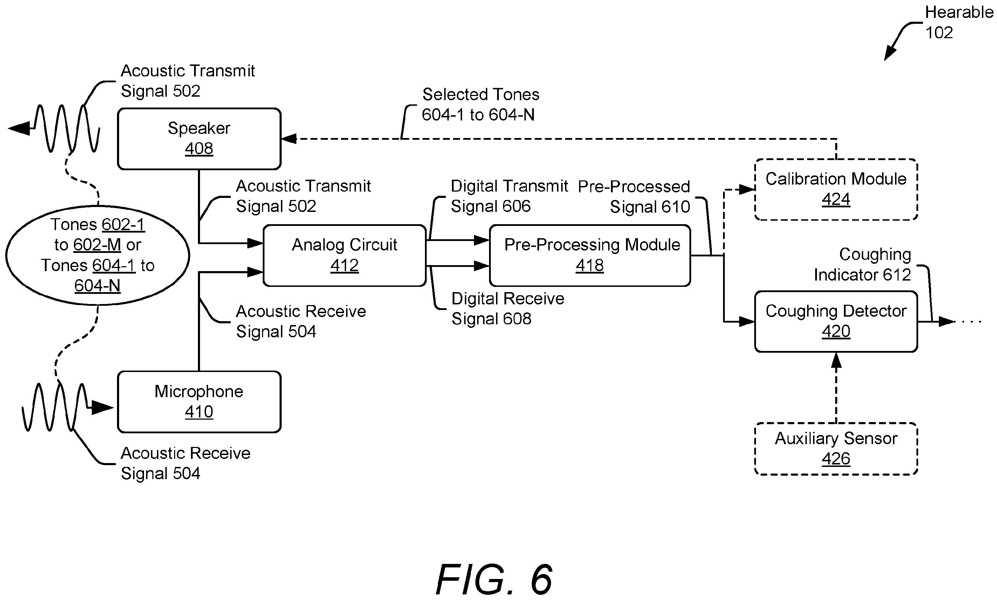

Operations of the pre-processing module 418, the coughing detector 420, and the calibration module 422 are further described with respect to FIG. 6. Aspects of user-focused coughing detection 112 using active acoustic sensing can be performed, at least partially, by the coughing detector 420.

Some hearables 102 include an active-noise-cancellation circuit 424, which enables the hearables 102 to reduce background or environmental noise. In this case, the microphone 410 used for audioplethysmography 110 can be implemented using a feedback microphone of the active-noise-cancellation circuit 424. During active noise cancellation, the feedback microphone provides feedback information regarding the performance of the active noise cancellation. During audioplethysmography 110, the feedback microphone receives an acoustic signal, which is provided to the pre-processing module 418. In some situations, active noise cancellation and audioplethysmography 110 are performed simultaneously using the feedback microphone. In this case, the acoustic signal received by the feedback microphone can be provided to the pre-processing module 418 and the feedback signal for active noise cancellation can be provided to the active-noise-cancellation circuit 424.

The hearable 102 can optionally include an auxiliary sensor 426. The auxiliary sensor 426 can have a different sensing modality than audioplethysmography 110 to assist with user-specific coughing detection 112. An example auxiliary sensor 426 can include a voice accelerometer or a microphone, which can detect coughing based on the over-the-air sound produced by the user 106's coughing.

Although not explicitly shown in FIG. 4, the system medium 416 can also include the application 306 (or another application). In this case, the hearable 102 uses the application 306 to perform one or more aspects of coughing monitoring and/or health monitoring. Different types of audioplethysmography 110 are further described with respect to FIG. 5.

### Active Acoustic Sensing

FIG. 5 illustrates example operations of two hearables 102-1 and 102-2. In a first example operation, the hearables 102-1 and 102-2 perform single-ear audioplethysmography 110. This means that the hearables 102-1 and 102-2 independently perform audioplethysmography 110 on different ears 108 of the user 106. In this case, the first hearable 102-1 is proximate to (arranged at) the user 106's right ear 108, and the second hearable 102-2 is proximate to (arranged at) the user 106's left ear 108. Each hearable 102-1 and 102-2 includes a speaker 408 and a microphone 410. The hearables 102-1 and 102-2 can operate in a monostatic manner during the same time period or during different time periods. In other words, each hearable 102-1 and 102-2 can independently transmit and receive acoustic signals.

For example, the first hearable 102-1 uses the speaker 408 to transmit a first acoustic transmit 502-1, which propagates within at least a portion of the user 106's right ear canal 114. The first hearable 102-1 uses the microphone 410 to receive a first acoustic receive signal 504-1. The first acoustic receive signal 504-1 represents a version of the first acoustic transmit signal 502-1 that is modified, at least in part, by the acoustic circuit associated with the right ear canal 114. This modification can change an amplitude, phase, and/or frequency of the first acoustic receive signal 504-1 relative to the first acoustic transmit signal 502-1.

Similarly, the second hearable 102-2 uses the speaker 408 to transmit a second acoustic transmit signal 502-2, which propagates within at least a portion of the user 106's left ear canal 114. The second hearable 102-2 uses the microphone 410 to receive a second acoustic receive signal 504-2. The second acoustic receive signal 504-2 represents a version of the second acoustic transmit signal 502-2 that is modified by the acoustic circuit associated with the left ear canal 114. This modification can change an amplitude, phase, and/or frequency of the second acoustic receive signal 504-2 relative to the second acoustic transmit signal 502-2.

The techniques of single-ear audioplethysmography 110 can be particularly beneficial as it enables the computing device 104 to compile information from both hearables 102-1 and 102-2, which can further improve measurement confidence. For some aspects of audioplethysmography 110, it can be beneficial to analyze the acoustic channel between two ears 108, as further described below.

In a second example operation, the two hearables 102-1 and 102-2 perform two-ear audioplethysmography 110. This means that the hearables 102-1 and 102-2 jointly perform audioplethysmography 110 across two ears 108 of the user 106. In this case, at least one of the hearables 102 (e.g., the first hearable 102-1) includes the speaker 408, and at least one of the other hearables 102 (e.g., the second hearable 102-2) includes the microphone 410. The hearables 102-1 and 102-2 operate together in a bistatic manner during the same time period.

During operation, the first hearable 102-1 transmits a third acoustic transmit 502-3 using the speaker 408. The third acoustic transmit signal 502-3 propagates through the user 106's right ear canal 114. The third acoustic transmit signal 502-3 also propagates through an acoustic channel that exists between the right and left ears 108. In the left ear 108, the third acoustic transmit signal 502-3 propagates through the user 106's left ear canal 114 and is represented as a third acoustic receive signal 504-3. The second hearable 102-2 receives the third acoustic receive signal 504-3 using the microphone 410. The third acoustic receive signal 504-3 represents a version of the third acoustic transmit signal 502-3 that is modified by the acoustic circuit associated with the right ear canal 114, modified by the acoustic channel associated with the user 106's face, and modified by the acoustic circuit associated with the left ear canal 114. This modification can change an amplitude, phase, and/or frequency of the third acoustic receive signal 504-3 relative to the third acoustic transmit signal 502-3. In some cases, the hearable 102-2 measures the time-of-flight (ToF) associated with the propagation from the first hearable 102-1 to the second hearable 102-2. Sometimes a combination of single-ear and two-ear audioplethysmography 110 are applied to further improve measurement confidence.

The acoustic transmit signals 502 of FIG. 5 can represent a variety of different types of signals as described above with respect to FIG. 4. In example implementations, the acoustic transmit signal 502 can be a continuous-wave signal (e.g., a sinusoidal signal) or a pulsed signal. Some acoustic transmit signals 502 can have a particular tone (or frequency). Other acoustic transmit signals 502 can have multiple tones (or multiple frequencies). A variety of modulations can be applied to generate the acoustic transmit signal 502. Example modulations include linear frequency modulations, triangular frequency modulations, stepped frequency modulations, phase modulations, or amplitude modulations. The acoustic transmit signal 502 can be transmitted as part of a calibration procedure or a measurement procedure, as further described as part of FIG. 6.

### User-Focused Coughing Detection

FIG. 6 illustrates an example implementation of the hearable 102. In the depicted configuration, the hearable 102 includes the speaker 408, the microphone 410, the analog circuit 412, the pre-processing module 418, the coughing detector 420, and the calibration module 422. Other implementations of the hearable 102 are also possible in which the hearable 102 does not include the calibration module 422 to reduce processing power requirements. In this case, the pre-processing module 418 can perform aspects of frequency selection as further described below to improve the signal-to-noise ratio for audioplethysmography 110. Some implementations of the hearable 102 can also include the auxiliary sensor 426. The auxiliary sensor 426 can be a separate sensor, such as a voice accelerometer, or can represent the microphone 410.

Outputs of the speaker 408 and the microphone 410 are coupled to inputs of the analog circuit 412. The pre-processing module 418 has inputs that are coupled to outputs of the analog circuit 412. The pre-processing module 418 also has an output that is coupled to inputs of the coughing detector 420 and the calibration module 422. In an example implementation, the pre-processing module 418 includes at least one in-phase and quadrature mixer (I/Q mixer) and at least one filter. The in-phase and quadrature mixer performs frequency down-conversion and can be implemented using at least two mixers, at least one phase shifter, and at least one combiner (e.g., a summation circuit). The filter attenuates intermodulation products that are generated by the in-phase and quadrature mixer. In an example implementation, the filter is implemented using a low-pass filter.

The pre-processing module 418 can optionally include at least one frequency selector. The frequency selector can identify and select one or more tones (or carrier frequencies) that provide a high-quality signal for later processing. The frequency selector can further pass the selected tones to other processing modules (e.g., the coughing detector 420) and filter (or attenuate) other tones that are not selected. The frequency selector can be implemented in a similar manner as the calibration module 422, which is further described below.

An output of the coughing detector 420 can be passed to the computing device 104, the application 306, or another process that is performed by the hearable 102. The coughing detector 420 can be implemented using a variety of techniques, including signal processing algorithms and/or a machine-learned model. With the coughing detector 420, the hearable 102 performs a measurement procedure that includes performing user-focused coughing detection 112 using audioplethysmography 110. **In** some implementations, the coughing detector 420 can perform some aspect of user-focused coughing detection 112 based on additional information provided by the auxiliary sensor 426.

The calibration module 422 has an output that is coupled to the speaker 408. The calibration module 422 includes at least one frequency selector. The frequency selector can include at least one amplitude detector, at least one phase detector, at least one quality detector, and at least one comparator. Using the frequency selector, the calibration module 422 can perform a calibration procedure that determines appropriate characteristics (e.g., waveform or signal characteristics) of acoustic transmit signals 502 to improve audioplethysmography 110 (e.g., to enhance the performance of user-focused coughing detection 112). The calibration procedure enables audioplethysmography 110 to take into account the wear of the hearable 102 (e.g., the position of the hearable 102 relative to the ear canal 114) and the physical structure of the ear canal 114 to determine a transmission frequency that can increase sensitivity.

Consider an example operation of the hearable 102 in accordance with single-ear audioplethysmography 110. In this example, the hearable 102 includes the calibration module 422. With the calibration module 422, the hearable 102 can perform a calibration procedure prior to performing a measurement procedure. In some circumstances, the hearable 102 can perform on-head detection (or in-ear detection) by detecting the presence of the seal 116 and initiating the calibration procedure and/or the measurement procedure based on a determination that on-head detection is "true." In other circumstances, the hearable 102 can initiate the calibration procedure based on a specified schedule or a timer, which can be controlled by the user 106 via the computing device 104 or the hearable 102. The calibration procedure and the measurement procedure are further described below.

During both the calibration procedure and the measurement procedure, the speaker 408 transmits the acoustic transmit signal 502 and the microphone 410 receives the acoustic receive signal 504. During the calibration procedure, the acoustic transmit signal 502 and the acoustic receive signal 504 can have tones 602-1 to 602-M, where M represents a positive integer. The multiple tones 602-1 to 602-M can be transmitted in parallel or in series over a given time interval. In this case, the acoustic transmit signal 502 can have a particular bandwidth on the order of several kilohertz. For example, the acoustic transmit signal 502 can have a bandwidth of approximately 4, 5, 6, 8, 10, 16, or 20 kHz. In example implementations, the acoustic transmit signal 502 is transmitted over multiple seconds, such as 2, 3, 4, 6, or more seconds. A duration of each tone 602 can be evenly divided over a total duration of the acoustic transmit signal 502.

In an example implementation, the acoustic transmit signal 502 for the calibration procedure can have seven tones 602 (e.g., M equals 7). In some cases, the tones 602 are evenly distributed across an interval. For example, the tones 602 can be in 1 kHz increments between 32 kHz and 38 kHz (e.g., at approximately 32, 33, 34, 35, 36, 37, and 38 kHz). The term "approximately" means that the tones 602 can be within 5% of a given value or less (e.g., within 3%, 2%, or 1% of the given value).

An amplitude of the calibration procedure's acoustic transmit signal 502 can be approximately the same across the tones 602-1 to 602-M. In this manner, power is evenly distributed across each tone 602. The quantity of tones 602 (e.g., *M*) can be determined based on an output power of the speaker 408. Increasing the quantity of tones 602 can increase a likelihood that the hearable 102 can support user-focused coughing detection 112 across various conditions including user wear and a physical structure of the user 106's ear canal 114. However, an amplitude of the acoustic transmit signal 502 can be limited across these tones 602 based on the output power of the speaker 408. Thus, the quantity of tones 602 can be optimized based on an amount of output power that is available for audioplethysmography 110.

During the measurement procedure, the acoustic transmit signal 502 and the acoustic receive signal 504 can have selected tones 604-1 to 604-N, where *N* represents a positive integer that is less than or equal to M. The selected tones 604-1 to 604-N can represent a subset (sometimes a proper subset) of the tones 602-1 to 602-M. The selected tones 604 can be transmitted in parallel or in series over a given time interval.

An amplitude of the measurement procedure's acoustic transmit signal 502 can be approximately the same across the selected tones 604-1 to 604-N. In this manner, power is evenly distributed across each selected tone. The amplitude of the measurement procedure's acoustic transmit signal 502 can be higher than the amplitude of the calibration procedure's acoustic transmit signal 502 because the available output power is distributed across fewer tones. Additionally or alternatively, a duration of each of the selected tones 604 of the measurement procedure's acoustic transmit signal 502 can be longer than the duration of the tones 602 of the calibration procedure's acoustic transmit signal 502. The higher amplitude and/or the longer duration can further improve the signal-to-noise ratio performance of the hearable 102 for audioplethysmography 110. By using a few selected tones 604 that were determined to improve signal-to-noise ratio performance, the measurement procedure can achieve a higher level of accuracy and sensitivity for user-focused coughing detection 112.

The analog circuit 412 performs analog-to-digital conversion to generate a digital transmit signal 606 and a digital receive signal 608 based on the acoustic transmit signal 502 and the acoustic receive signal 504, respectively. The pre-processing module 418 performs frequency downconversion and demodulation to generate at least one pre-processed signal 610 based on the digital transmit signal 606 and the digital receive signal 608. The pre-processing module 418 can also apply filtering to generate the pre-processed signal 610.

Optionally, as part of the calibration procedure, the calibration module 422 processes the pre-processed signal 610 to determine the selected tones 604-1 to 604-N. The selected tones 604-1 to 604-N can improve performance of audioplethysmography 110 during the measurement procedure. To determine the selected tones 604-1 to 604-N, the calibration module 422 extracts the amplitude and/or phase of the pre-processed signal 610 using the amplitude detector and the phase detector, respectively. The quality detector of the calibration module 422 measures quality metrics for each tone (or frequency) of the pre-processed signal 610 and for each of the characteristics (e.g., amplitude and/or phase). Example quality metrics can include peak-to-average ratios and/or signal-to-noise ratios. The peak-to-average ratio represents a peak intensity within a frequency range of interest divided by an average intensity within this frequency range. A higher quality metric indicates a higher-quality signal, or more generally, better performance for audioplethysmography 110.

The comparator of the calibration module 422 can evaluate the quality metrics with respect to a threshold. In an example implementation, the comparator determines the selected tones 604-1 to 604-N for a subsequent measurement procedure based on the frequencies associated with the quality metrics that are greater than or equal to a threshold. Additionally or alternatively, the comparator can evaluate the quality metrics with respect to each other. In an example implementation, the comparator determines one of the selected tones based on a frequency with the highest quality metric across the amplitude. Also, the comparator can determine one of the selected tones 604-1 to 604-N based on a frequency with the highest quality metric across the phase. In other implementations, the comparator can determine a single selected tone based on a frequency having the highest quality metric associated with either the amplitude or the phase.

In general, the calibration module 422 enables the selected tones 604-1 to 604-N to be dynamically adjusted prior to the measurement procedure based on a current environment, which can account for a wear of the hearable 102 (e.g., a current insertion depth and/or rotation), a physical structure of the user 106's ear canal 114, and a response characteristic of the hearable 102 (e.g., speaker, microphone, and/or housing). In this manner, the calibration module 422 can improve the signal-to-noise ratio performance of the hearable 102 for the measurement procedure. The calibration module 422 can also determine which tones 604 generate acoustic receive signals 504 with desired characteristics for user-focused coughing detection 112. In general, the calibration procedure can be performed whether or not the user 106 is coughing.

The calibration module 422 communicates the selected tones 604-1 to 604-N to the speaker 408 using a control signal. The speaker 408 accepts the control signal that identifies the selected tones 604-1 to 604-N and can transmit a subsequent acoustic transmit signal 502 for user-focused coughing detection 112 using the selected tones 604-1 to 604-N. With the calibration procedure, the hearable 102 can dynamically adjust the transmission frequency (e.g., one or more carrier frequencies) each time the seal 116 is formed (e.g., based on the wear of the hearable 102) and based on the unique physical structure of the ear 108. Through this calibration procedure, the hearables 102 on different ears 108 may operate with one or more different acoustic frequencies (e.g., ultrasound frequencies).

As part of the measurement procedure, the coughing detector 420 can perform aspects of coughing detection 112 using the pre-processed signal 610 to generate a coughing indicator 612. In an example implementation, the coughing indicator 612 is a control signal having a first state that indicates coughing is detected and a second state that indicates coughing is not detected.

To perform user-focused coughing detection 112, the coughing detector 420 analyzes the pre-processed signal 610 and detects a significant variation in an amplitude and/or phase of the pre-processed signal 610. This variation is caused by the deformation in the ear canal 114 due to muscle movements and/or sound that is made by the user 106 as the user 106 coughs. Variations can also occur due to muscle movements that are made by the user 106 in anticipation of coughing. An example muscle movement can involve the user 106 opening their jaw in preparation to cough.

In general, the term "significantly" can mean that the values of the amplitude and/or the phase can change by 20% or more relative to a previous value (e.g., relative to an average of a set of previous values). Additionally or alternatively, a slope of the amplitude and/or the phase can vary significantly. Sometimes the slope of the amplitude and/or the phase can change signs (e.g., from a positive slope to a negative slope, or vice versa). A magnitude of the slope of the amplitude and/or the phase can sometimes change by approximately 10% or more.

The coughing detector 420 can be implemented in a variety of ways to detect the variation in the pre-processed signal 610. In a first method, the coughing detector 420 determines that the user 106 coughed based on a peak of the amplitude and/or phase exceeding a predetermined threshold. In a second method, the coughing detector 420 determines that the user 106 coughed based on a change in a slope of the amplitude and/or phase exceeds a predetermined threshold. In a third method, the coughing detector 420 computes a variance of the pre-processed signal 610 and determines that the user 106 coughed based on the variance exceeding a predetermined threshold.

In a fourth method, the coughing detector 420 uses both active acoustic sensing and the auxiliary sensor 426 to perform user-focused coughing detection 112. In this case, the coughing detector 420 processes the pre-processed signal 610 to determine whether active acoustic sensing detected the user 106 coughing. The coughing detector 420 also processes information provided by the auxiliary sensor 426 (e.g., a received over-the-air voice signal) to determine whether the auxiliary sensor 426 detected the user 106 coughing. If coughing is detected based on both the pre-processed signal 610 and the auxiliary sensor 426, the coughing detector 420 generates the coughing indicator 612 to indicate that the user 106 coughed. Otherwise, the coughing detector 420 generates the coughing indicator 612 to indicate that the user 106 did not cough.

With the auxiliary sensor 426, the hearable 102 can also detect when coughing that is not associated with the user 106 occurs in the external environment. For example, the hearable 102 can determine that another source (e.g., the other person 206) coughed if coughing is detected based on the information provided by the auxiliary sensor 426 but is not detected based on the pre-processed signal 610. This information can also be useful to indicate whether or not the user 106 may have been in proximity to someone else who was potentially ill.

In a fifth method, the coughing detector 420 uses a machine-learned model that is trained, using supervised learning, to detect a change in the pre-processed signal 610 that is associated with the user 106 coughing. The machine-learned model can be implemented using one or more neural networks. A neural network includes a group of connected nodes (e.g., neurons or perceptrons), which are organized into one or more layers. As an example, the machine-learned model includes a deep neural network, which includes an input layer, an output layer, and one or more hidden layers positioned between the input layer and the output layers. The nodes of the deep neural network can be partially-connected or fully-connected between the layers.

In some implementations, the neural network is a recurrent neural network (e.g., a long short-term memory (LSTM) neural network) with connections between nodes forming a cycle to retain information from a previous portion of an input data sequence for a subsequent portion of the input data sequence. In other cases, the neural network is a feed-forward neural network in which the connections between the nodes do not form a cycle. Additionally or alternatively, the machine-learned model includes another type of neural network, such as a convolutional neural network. The machine-learned model can also include one or more types of classification models, such as a binary classification model, a multi-class classification model, multi-label classification, and so forth.

In example implementations, the machine-learned model can be implemented using a single-channel-input machine-learned model or a multi-channel-input machine-learned model. The single-channel-input machine-learned model performs user-focused coughing detection 112 based on the pre-processed signal 610. The multi-channel-input machine-learned model performs user-focused coughing detection 112 based on the pre-processed signal 610 and information provided by the auxiliary sensor 426. Additionally or alternatively, the multi-channel-input machine-learned model can perform user-focused coughing detection 112 based on multiple pre-processed signals 610 provided by different hearables 102 (e.g., the hearables 102-1 and 102-2 of FIG. 5).

In general, the machine-learned model is trained using supervised learning to generate the coughing indicator 612 based on at least a version of the acoustic receive signal 504. The supervised learning can use simulated (e.g., synthetic) data or measured (e.g., real) data for training purposes. In some implementations, the machine-learned model is further trained to identify the different types of coughs.

In FIG. 6, the calibration procedure and the measurement procedure are described as individual procedures that occur at different time intervals. In particular, the calibration procedure occurs before the measurement procedure. This enables the acoustic transmit signal 502 for the measurement procedure to be transmitted with fewer tones than the acoustic transmit signal 502 used for the calibration procedure, which can increase signal-to-noise ratio performance for audioplethysmography 110. In some implementations, however, the hearable 102 can have sufficient output power to perform the measurement procedure with the multiple tones 602-1 to 602-M using a single acoustic transmit signal 502. In this case, aspects of the calibration module 422 can be integrated within the pre-processing module 418 via a frequency selector. This frequency selector can effectively pass the selected tones 604-1 to 604-N to the coughing detector 420.

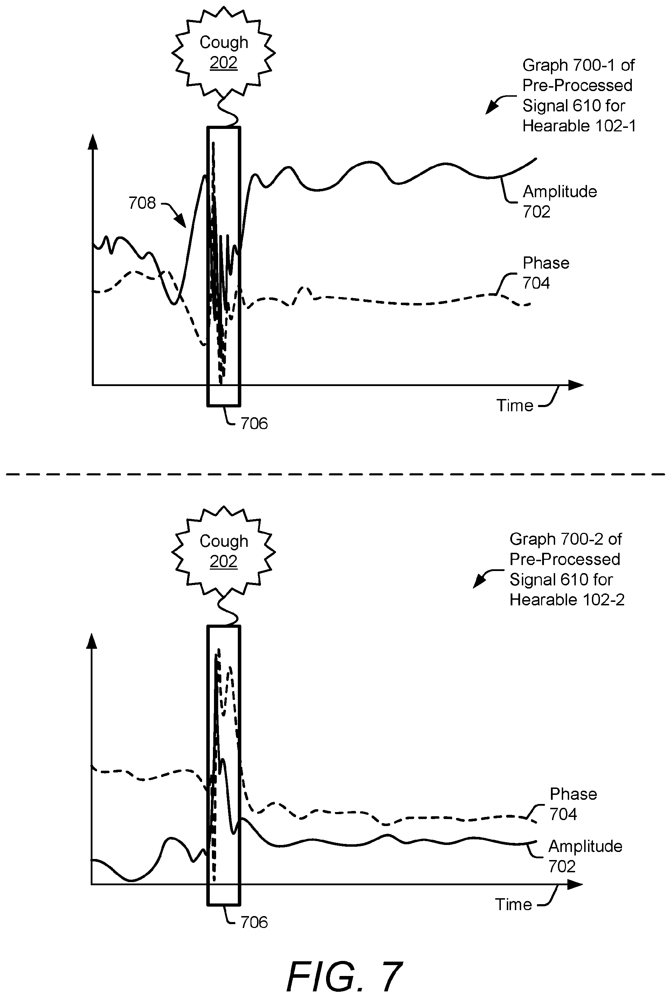

FIG. 7 illustrates an impact of coughing on an acoustic receive signal 504. More specifically, FIG. 7 depicts example amplitudes and phases of pre-processed signals 610 generated by different hearables 102-1 and 102-2. As shown below, the pressure wave caused by the user 106 coughing can significantly impact the amplitude and/or the phase of the pre-processed signals 610. In some instances, the change in the amplitude and/or the phase can be relative to a previous state or relative to a previous trend in the amplitude and/or the phase. The previous state can refer to values of the amplitude and/or the phase during which the user 106 does not cough.

In some implementations, the coughing detector 420 can detect and recognize an intention of the user 106 to cough as well as the action of the user 106 coughing based on the amplitude of the pre-processed signal 610 provided by the hearable 102-1, the phase of the pre-processed signal 610 provided by the hearable 102-1, the amplitude of the pre-processed signal 610 provided by the hearable 102-2, the phase of the pre-processed signal 610 provided by the hearable 102-2, or some combination thereof. Generally speaking, processing a larger quantity of signals and/or tones 604 provides more information to the coughing detector 420. This can make it easier for the coughing detector 420 to accurately perform user-focused coughing detection 112.

Graphs 700-1 and 700-2 in FIG. 7 depict amplitudes 702 and phases 704 of pre-processed signals 610 that are respectively generated by the hearables 102-1 and 102-2. Time is depicted along the horizontal axes of the graphs 700-1 and 700-2. During the time interval indicated at 706, the user 106 coughs 202. This causes the amplitude 702 and/or the phase 704 of the acoustic receive signal 504 to change significantly relative to a previous state.

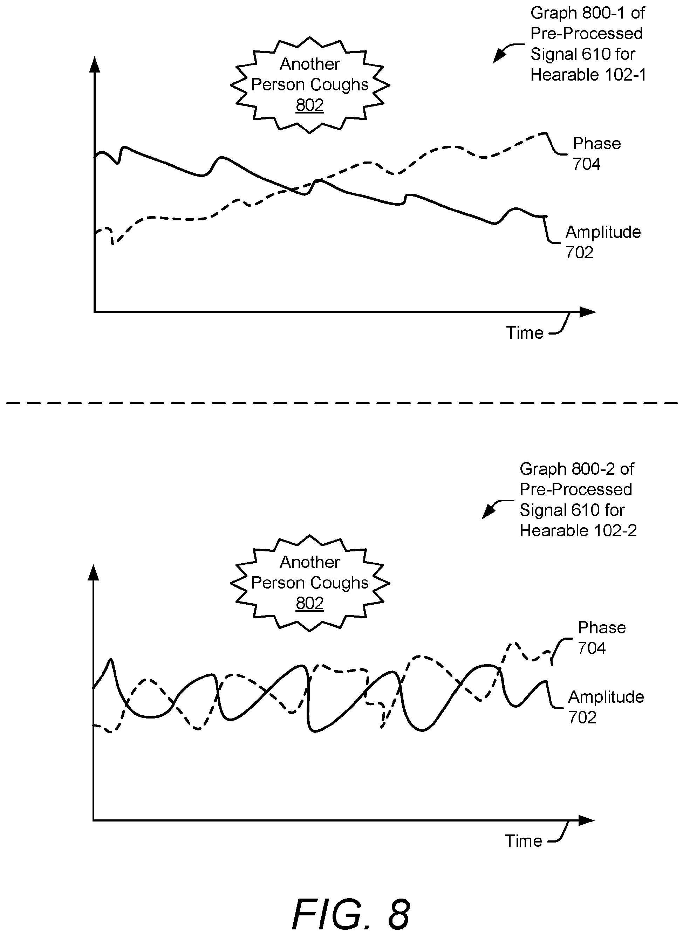

Prior to time 706, as indicated by 708, the user 106 makes muscle movements in anticipation of coughing. These muscle movements can include moving their jaw (e.g., opening their jaw). With audioplethysmography 110, the coughing detector 420 can detect and recognize the intention of the user 106 to cough at 708 based on the change in the amplitude 702 and/or phase 704 of the pre-processed signals 610 provided by the hearable 102-1 and/or the hearable 102-2. Sometimes, as shown in FIG. 7, one of the two hearables 102-1 and 102-2 can be used to detect the intention of the user 106 to cough. In this example, the pre-processed signal 610 provided by the hearable 102-1 can be used to detect and recognize the intention of the user 106 to cough whereas the pre-processed signal 610 provided by the hearable 102-2 does not include this information. These significant fluctuations indicated at 706 and 708 are absent during other time periods in which the user 106 does not cough and another person 206 proximate to the hearable 102 coughs, as further described with respect to FIG. 8.

FIG. 8 illustrates an impact of another person 206 coughing on an acoustic receive signal 504. Graphs 800-1 and 800-2 in FIG. 8 depict amplitudes 702 and phases 704 of pre-processed signals 610 that are respectively generated by the hearables 102-1 and 102-2. Time is depicted along the horizontal axes of the graphs 800-1 and 800-2. During the time interval depicted, the user 106 does not cough 202 but another person 206 that is proximate to the hearable 102 (and who is not wearing the hearable 102) coughs 202, as indicated by 802.

As shown in the graphs 800-1 and 800-2, the coughing of the other person 206 does not significantly change the amplitude 702 and/or phase 704 of the pre-processed signals 610. This is because the person 206's cough 202 minimally affects the user 106's ear canal 114. As such, active acoustic sensing enables user-focused coughing detection 112 by reducing the risk of false positives caused by the other person 206 coughing.

A scaling of the amplitudes 702 and the phases 704 may not necessarily be the same across the graphs 700-1, 700-2, 800-1 and 800-2. In general, an impact of the user 106 coughing can cause variations in the pre-processed signal 610 to be at least two times greater than variations observed while the user 106 is not coughing or variations observed while the other person 206 is coughing. In many cases, the impact of the user 106 coughing can cause variations in the pre-processed signal 610 to be at least ten times greater than variations observed while the user 106 is not coughing or variations observed while the other person 206 is coughing.

Aspects of user-focused coughing detection 112 can be performed using one hearable 102 (e.g., the hearable 102-1 or 102-2) or multiple hearables 102 (e.g., the hearables 102-1 and 102-2). With multiple hearables 102 performing coughing detection 112, the computing device 104 can have higher confidence that the user 106's cough 202 is detected. In general, the hearable 102 can detect coughing by analyzing changes in the amplitude of the acoustic receive signal 504, changes in the phase of the acoustic receive signal 504, or changes in both the amplitude and phase of the acoustic receive signal 504.

### Example Methods

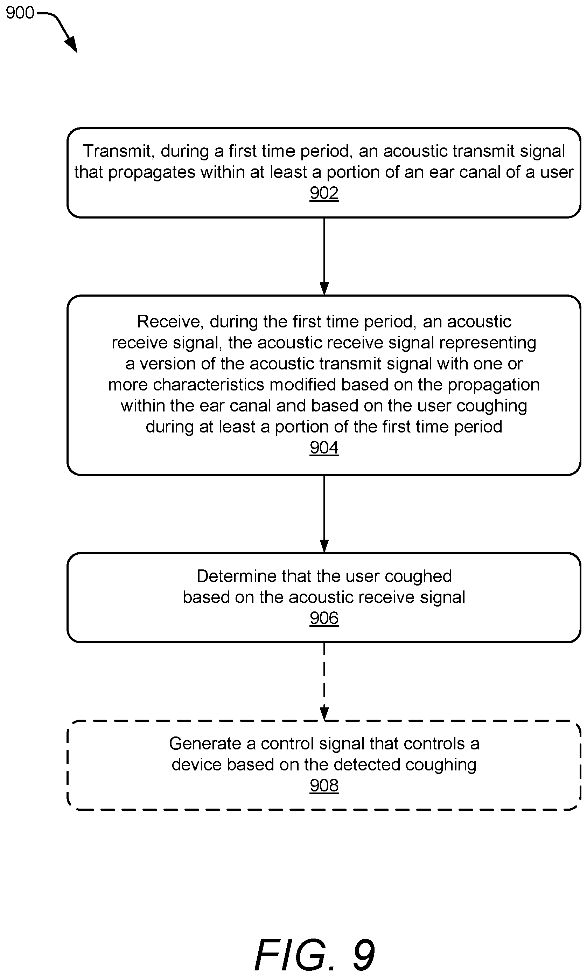

FIGs. 9, 10, and 11 depict example methods 900, 1000, and 1100 for implementing aspects of user-focused coughing detection 112 using active acoustic sensing. Methods 900, 1000, and 1100 are shown as sets of operations (or acts) performed but not necessarily limited to the order or combinations in which the operations are shown herein. Further, any of one or more of the operations may be repeated, combined, reorganized, or linked to provide a wide array of additional and/or alternate methods. In portions of the following discussion, reference may be made to the environments 100, 200-1, or 200-2 of FIGs. 1 or 2, and entities detailed in FIGs. 3 and 4, reference to which is made for example only. The techniques are not limited to performance by one entity or multiple entities operating on one device.

At 902, an acoustic transmit signal is transmitted during a first time period. The acoustic transmit signal propagates within at least a portion of an ear canal of a user. For example, the transducer 406 (or speaker 408) of the hearable 102 transmits the acoustic transmit signal 502. The acoustic transmit signal 502 propagates within at least a portion of the ear canal 114 of the user 106, as described with respect to FIG. 5.

At 904, an acoustic receive signal is received. The acoustic receive signal represents a version of the acoustic transmit signal with one or more characteristics (e.g., waveform characteristics) modified based on the propagation within the ear canal and based on the user coughing during at least a portion of the first time period. For example, the transducer 406 (or the microphone 410) of the hearable 102 receives the acoustic receive signal 504. The acoustic receive signal 504 represents a version of the acoustic transmit signal 502 with one or more characteristics modified based on the propagation within the ear canal 114 and based on the user 106 coughing during at least a portion of the first time period. More specifically, the acoustic receive signal 504 is modulated based on the deformation that occurs within the ear canal 114 as caused by the user 106 coughing.

The hearable 102 that receives the acoustic receive signal 504 can be a same hearable 102 that transmitted the acoustic transmit signal 502 (e.g., the hearable 102-1 or 102-2 in FIG. 5), or another hearable 102 that did not transmit the acoustic transmit signal 502 (e.g., the hearable 102-2 in FIG. 5). Example characteristics include amplitude, phase, and/or frequency. In some implementations, a feedback microphone of an active-noise-cancellation circuit 424 can receive the acoustic receive signal 504.

At 906, the user is determined to have coughed based on the acoustic receive signal For example, the coughing detector 420 determines that the user 106 coughed based on the acoustic receive signal 504 (or more specifically based on the modified characteristics of the acoustic receive signal 504). The coughing detector 420 can determine that the user 106 coughed based on a change in the amplitude and/or phase of a signal that is derived from the acoustic receive signal 504 (e.g., based on the pre-processed signal 610).

Optionally at 908, a control signal that controls a device based on the detected coughing is generated. For example, the coughing detector 420 generates the coughing indicator 612. The coughing indicator 612 can be passed to a device to control an operation of the device. The device can include the computing device 104 and/or the hearable 102. Example controls can cause the device to update information regarding health monitoring, such as how often the user 106 coughs, when the user 106 coughs, and so forth. With this feedback, the user 106 can review their coughing history and determine whether or not to take medication or seek medical attention. In addition to assisting the user 106 with monitoring their health, the device 104 can use information associated with user-focused coughing detection 112 to perform early detection warning of a potential illness.

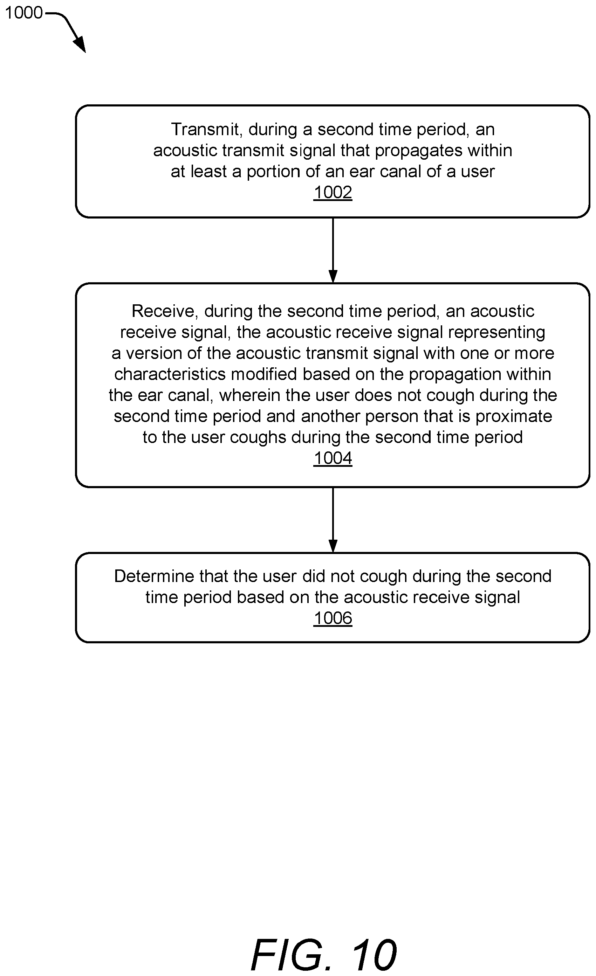

At 1002 in FIG. 10, an acoustic transmit signal is transmitted during a second time period. The acoustic transmit signal propagates within at least a portion of an ear canal of a user. For example, the transducer 406 (or speaker 408) of the hearable 102 transmits the acoustic transmit signal 502. The acoustic transmit signal 502 propagates within at least a portion of the ear canal 114 of the user 106, as described with respect to FIG. 5.

At 1004, an acoustic receive signal is received during the second time period. The acoustic receive signal represents a version of the acoustic transmit signal with one or more characteristics (e.g., waveform characteristics) modified based on the propagation within the ear canal. During the second time period, the user does not cough and another person that is proximate to the user coughs. For example, the transducer 406 (or the microphone 410) of the hearable 102 receives the acoustic receive signal 504 during the second time period. The acoustic receive signal 504 represents a version of the acoustic transmit signal 502 with one or more characteristics modified based on the propagation within the ear canal 114. During the second time period, the user 106 does not cough and another person 206 that is proximate to the user 106 coughs. The term "proximate" means that the other person 206 can be close enough to the user 106 that the user 106 can hear the other person 206 coughing. Additionally or alternatively, the term "proximate" can mean that the other person 206 is sufficiently close to the hearable 102 that a microphone of the hearable 102 can detect the other person 206 coughing. Additionally or alternatively, the term "proximate" can mean that the other person 206 (no wearing the hearable 102) is located within a distance of more than 30 centimeters and less than 10 meters, in particular less than 5 meters to the hearable 102.

At 1006, the user is determined to not have coughed during the second time period based on the acoustic receive signal. For example, the coughing detector 420 determined that the user 106 did not cough during the second time period based on the acoustic receive signal 504 (e.g., based a signal derived from the acoustic receive signal 504). In this manner, user-focused coughing detection 112 can accurately and reliably discriminate between the user 106 coughing and another external source (e.g., another person 206 or media) producing coughing sounds. Optionally, the hearable 102 can determine that an external source produced the coughing sound if the information provided by the auxiliary sensor 426 indicates the presence of coughing during the second time period.

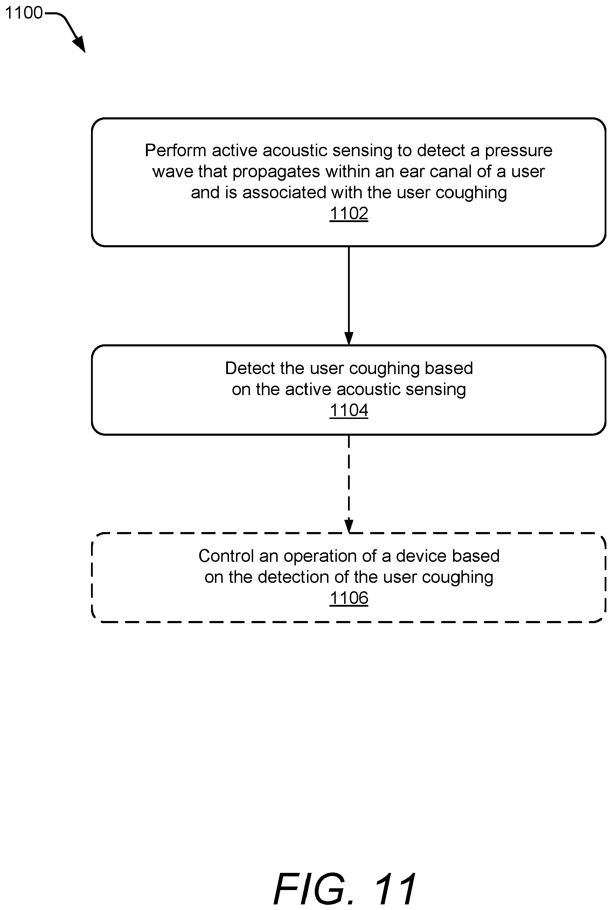

At 1102 in FIG. 11, active acoustic sensing is performed to detect a pressure wave that propagates to an ear canal of a user and is associated with the user coughing. For example, the hearable 102 performs active acoustic sensing to detect the pressure wave that propagates to the ear canal 114 of the user 106 and is associated with the user 106 coughing. More specifically, the hearable 102 transmits and receives an acoustic signal during the first time period. The acoustic signal propagates within at least a portion of the ear canal 114 of the user 106. The received acoustic signal (e.g., the acoustic receive signal 504) represents a version of the transmitted acoustic signal (e.g., the acoustic transmit signal 502) with one or more characteristics (e.g., amplitude 702 and/or phase 704) modified based on the propagation within the ear canal 114 and based on the user 106 coughing during at least a portion of the first time period.

At 1104, the coughing is detected based on the active acoustic sensing. For example, the coughing detector 420 performs user-focused coughing detection 112 based on the active acoustic sensing (e.g., based on the version of the acoustic receive signal 504, such as the pre-processed signal 610). Optionally, the coughing detector 420 performs user-focused coughing detection 112 based on the active acoustic sensing and based on the information provided by an auxiliary sensor, such as a voice accelerometer or a microphone. In some implementations, the type of coughing can also be determined based on the active acoustic sensing and/or the auxiliary sensor.

At 1106, an operation of a device is controlled based on the detection of the user coughing. For example the coughing detector 420 generates a coughing indicator 612, which can control an operation of the hearable 102 and/or the computing device 104.

### Example Computing System

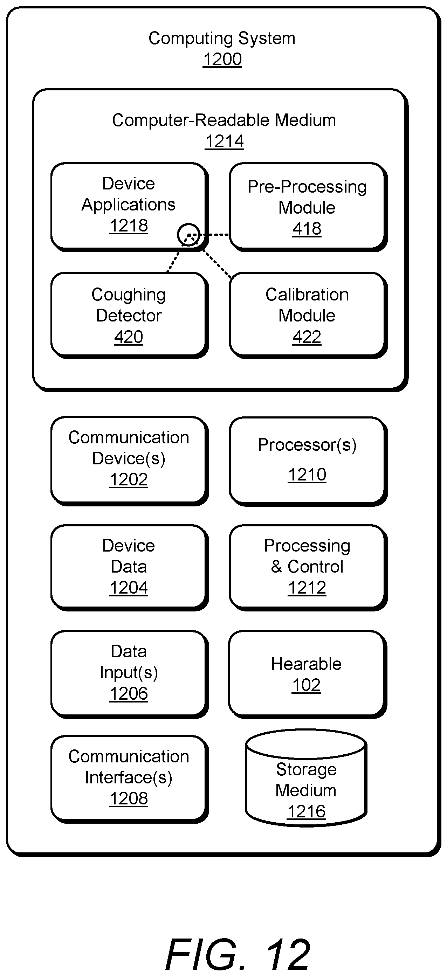

FIG. 12 illustrates various components of an example computing system 1200 that can be implemented as any type of client, server, and/or computing device as described with reference to the previous FIGs. 3 and 4 to implement aspects of active acoustic sensing using a hearable 102. The computing system 1200 includes communication devices 1202 that enable wired and/or wireless communication of device data 1204 (e.g., received data, data that is being received, data scheduled for broadcast, or data packets of the data). The communication devices 1202 or the computing system 1200 can include one or more hearables 102. The device data 1204 or other device content can include configuration settings of the device, media content stored on the device, and/or information associated with a user of the device. Media content stored on the computing system 1200 can include any type of audio, video, and/or image data. The computing system 1200 includes one or more data inputs 1206 via which any type of data, media content, and/or inputs can be received, such as human utterances, user-selectable inputs (explicit or implicit), messages, music, television media content, recorded video content, and any other type of audio, video, and/or image data received from any content and/or data source.

The computing system 1200 also includes communication interfaces 1208, which can be implemented as any one or more of a serial and/or parallel interface, a wireless interface, any type of network interface, a modem, and as any other type of communication interface. The communication interfaces 1208 provide a connection and/or communication links between the computing system 1200 and a communication network by which other electronic, computing, and communication devices communicate data with the computing system 1200.

The computing system 1200 includes one or more processors 1210 (e.g., any of microprocessors, controllers, and the like), which process various computer-executable instructions to control the operation of the computing system 1200. Alternatively or in addition, the computing system 1200 can be implemented with any one or combination of hardware, firmware, or fixed logic circuitry that is implemented in connection with processing and control circuits which are generally identified at 1212. Although not shown, the computing system 1200 can include a system bus or data transfer system that couples the various components within the device. A system bus can include any one or combination of different bus structures, such as a memory bus or memory controller, a peripheral bus, a universal serial bus, and/or a processor or local bus that utilizes any of a variety of bus architectures.

The computing system 1200 also includes a computer-readable medium 1214, such as one or more memory devices that enable persistent and/or non-transitory data storage (i.e., in contrast to mere signal transmission), examples of which include random access memory (RAM), non-volatile memory (e.g., any one or more of a read-only memory (ROM), flash memory, EPROM, EEPROM, etc.), and a disk storage device. The disk storage device may be implemented as any type of magnetic or optical storage device, such as a hard disk drive, a recordable and/or rewriteable compact disc (CD), any type of a digital versatile disc (DVD), and the like. The computing system 1200 can also include a mass storage medium device (storage medium) 1216.

The computer-readable medium 1214 provides data storage mechanisms to store the device data 1204, as well as various device applications 1218 and any other types of information and/or data related to operational aspects of the computing system 1200. For example, an operating system 1220 can be maintained as a computer application with the computer-readable medium 1214 and executed on the processors 1210. The device applications 1218 may include a device manager, such as any form of a control application, software application, signal-processing and control module, code that is native to a particular device, a hardware abstraction layer for a particular device, and so on.

The device applications 1218 also include any system components, engines, or managers to implement audioplethysmography 110 for user-focused coughing detection 112. In this example, the device applications 1218 include the pre-processing module 418, the coughing detector 420, and optionally includes the calibration module 422. Although not explicitly shown, the device applications 1218 can also include the application 306.

Throughout this disclosure, examples are described where a computing system 1200 (e.g., the hearable 102, the computing device 104, a client device, a server device, a computer, or another type of computing system) may analyze information (e.g., various audible and/or acoustic signals) associated with a user 106, for example, the pre-processed signal 610 mentioned with respect to FIG. 6. Further to the descriptions above, a user 106 may be provided with controls allowing the user 106 to make an election as to both if and when systems, programs, and/or features described herein may enable collection of information (e.g., information associated with user-focused coughing detection 112, information about the user 106's health, information about the user 106's biometrics, information about a user 106's social network, social actions, social activities, profession, a user 106's preferences, a user 106's current location), and if the user 106 is sent content or communications from a server. The computing system 1200 can be configured to only use the information after the computing system 1200 receives explicit permission from the user 106 to use the data. For example, in situations where the hearable 102 analyzes signals for user-focused coughing detection 112, individual users 106 may be provided with an opportunity to provide input to control whether programs or features of the computing system 1200 can collect and make use of the data. Further, individual users 106 may have constant control over what programs can or cannot do with the information.

In addition, information collected may be pre-treated in one or more ways before it is transferred, stored, or otherwise used, so that personally-identifiable information is removed. For example, before the computing system 1200 shares data with another device, a user 106's identity may be treated so that no personally identifiable information can be determined for the user 106. Thus, the user 106 may have control over whether information is collected about the user 106 and the user 106's device, and how such information, if collected, may be used by the computing system 1200 and/or a remote computing system.

### Conclusion

Although techniques using, and apparatuses including, user-focused coughing detection using active acoustic sensing have been described in language specific to features and/or methods, it is to be understood that the scope of the invention is not necessarily limited to the specific features or methods described and is defined by the appended claims. Rather, the specific features and methods are disclosed as example implementations of user-focused coughing detection using active acoustic sensing.

## Claims

1. A method comprising:
transmitting (902), using a hearable (102; 102-1), during a first time period, an acoustic transmit signal (502; 502-1) that propagates within at least a portion of an ear canal (114) of a user (106);
receiving (904), using the hearable (102; 102-1), during the first time period, an acoustic receive signal (504; 504-1), the acoustic receive signal (504; 504-1) representing a version of the acoustic transmit signal (502; 502-1) with one or more characteristics modified due to the propagation within the ear canal (114) and based on the user (106) coughing during at least a portion of the first time period; and
determining (906) that the user (106) coughed during the first time period based on changes in an amplitude and/or a phase of the acoustic receive signal (504;504-1) due to the deformation of the ear canal (114).

2. The method of claim 1, further comprising:
generating a control signal that controls an operation of a device based on the determination that the user coughed.

3. The method of claim 2, wherein:
the device comprises the hearable (102; 102-1).

4. The method of claim 2, wherein:
the device comprises a computing device (104) that is coupled to the hearable (102; 102-1);
the transmitting of the acoustic transmit signal (502; 502-1) comprises transmitting the acoustic transmit signal (502; 502-1) using the hearable (102; 102-1); and
the receiving of the acoustic receive signal (504; 504-1) comprises receiving the acoustic receive signal (504; 504-1) using the hearable (102; 102-1).

5. The method of claim 4, wherein generating the control signal causes the computing device (104) to perform health monitoring based on the determination that the user (106) coughed.

6. The method of any previous claim, further comprising:
transmitting (1002), during a second time period, another acoustic transmit signal (502) that propagates within at least a portion of the ear canal (114) of the user (106);
receiving (1004), during the second time period, another acoustic receive signal (504), wherein the user (106) does not cough during the second time period and another person coughs during at least a portion of the second time period, the other person being proximate to the user; and
determining that the user (106) did not cough during the second time period based on an amplitude and/or a phase of the other acoustic receive signal (504) not being significantly changed.

7. The method of claim 6, further comprising:
sensing , using an auxiliary sensor (426) and during the second time period, an over-the-air voice signal produced by the other person coughing; and
determining that the other person coughed during the second time period based on the other acoustic receive signal and based on the sensing of the over-the-air voice signal.

8. The method of claim 7, further comprising:
sensing, using the auxiliary sensor (426) and during the first time period, another over-the-air voice signal produced by the user coughing,
wherein the determining that the user (106) coughed comprises determining that the user (106) coughed based on the acoustic receive signal (504) and based on the sensing of the other over-the-air voice signal.

9. The method of any previous claim, further comprising:
transmitting, using a further hearable (102-2), during the first time period, a second acoustic transmit signal (502-2) that propagates within at least a portion of a second ear canal (114) of the user (106); and
receiving, during the first time period, a second acoustic receive signal (504-2), the second acoustic receive signal (504-2) representing a version of the second acoustic transmit signal (502-2) with one or more characteristics modified due to the propagation within the second ear canal (114) and based on the user (106) coughing during at least a portion of the first time period,
wherein determining that the user (106) coughed comprises determining that the user (106) coughed during the first time period based on the acoustic receive signal (504-1) and the second acoustic receive signal (504-2).

10. A non-transitory computer-readable storage medium (1214) comprising instructions that, responsive to execution by a processor (1210), cause a hearable (102; 102-1, 102-2) to perform any one of the methods of claims 1 to 9.

11. A system comprising:
at least one hearable having at least one transducer (406); and
at least one processor (414), the system configured to perform, using the at least one transducer (406) and the at least one processor (414), any one of the methods of claims 1 to 9.

12. The system of claim 11, further comprising:
a speaker (408); and
an active-noise-cancellation circuit (424) comprising a feedback microphone (410),
wherein the at least one transducer (406) comprises the speaker (408) and the feedback microphone (410).

13. The system of claim 11, comprising a second hearable, wherein:
the at least one transducer (406) comprises a speaker (408) and a microphone (410);
the speaker (408) is configured to be positioned at a first ear (108) of a user (106); and
the microphone (410) is configured to be positioned at a second ear (108) of the user (106).

14. The system of any one of claims 11 to 13, wherein the at least one hearable (402-1, 402-2) comprises the at least one processor (414).

## Patentansprüche

1. Verfahren, umfassend:
Senden (902), unter Verwendung eines Hearables (102; 102-1), während eines ersten Zeitraums, eines akustischen Sendesignals (502; 502-1), das sich innerhalb zumindest eines Teils eines Gehörgangs (114) eines Benutzers (106) ausbreitet;
Empfangen (904), unter Verwendung des Hearables (102; 102-1), während des ersten Zeitraums, eines akustischen Empfangssignals (504; 504-1), wobei das akustische Empfangssignal (504; 504-1) eine Version des akustischen Sendesignals (502; 502-1) darstellt, bei der eine oder mehrere Merkmale aufgrund der Ausbreitung innerhalb des Gehörgangs (114) und basierend darauf, dass der Benutzer (106) während zumindest eines Abschnitts des ersten Zeitraums hustet, modifiziert sind; und
Bestimmen (906), dass der Benutzer (106) während des ersten Zeitraums gehustet hat, basierend auf Änderungen einer Amplitude und/oder einer Phase des akustischen Empfangssignals (504; 504-1) aufgrund der Verformung des Gehörgangs (114).

2. Verfahren nach Anspruch 1, ferner umfassend:
Erzeugen eines Steuersignals, das einen Betrieb einer Vorrichtung basierend auf der Bestimmung steuert, dass der Benutzer gehustet hat.

3. Verfahren nach Anspruch 2, wobei:
die Vorrichtung das Hearable (102; 102-1) umfasst.

4. Verfahren nach Anspruch 2, wobei:
die Vorrichtung eine Rechenvorrichtung (104) umfasst, die mit dem Hearable (102; 102-1) gekoppelt ist;
das Senden des akustischen Sendesignals (502; 502-1) das Senden des akustischen Sendesignals (502; 502-1) unter Verwendung des Hearables (102; 102-1) umfasst; und
das Empfangen des akustischen Empfangssignals (504; 504-1) das Empfangen des akustischen Empfangssignals (504; 504-1) unter Verwendung des Hearables (102; 102-1) umfasst.

5. Verfahren nach Anspruch 4, wobei das Erzeugen des Steuersignals die Rechenvorrichtung (104) veranlasst, eine Gesundheitsüberwachung basierend auf der Bestimmung durchzuführen, dass der Benutzer (106) gehustet hat.

6. Verfahren nach einem der vorhergehenden Ansprüche, ferner umfassend:
Senden (1002), während eines zweiten Zeitraums, eines weiteren akustischen Sendesignals (502), das sich innerhalb zumindest eines Abschnitts des Gehörgangs (114) des Benutzers (106) ausbreitet;
Empfangen (1004), während des zweiten Zeitraums, eines weiteren akustischen Empfangssignals (504), wobei der Benutzer (106) während des zweiten Zeitraums nicht hustet und eine andere Person während zumindest eines Abschnitts des zweiten Zeitraums hustet, wobei sich die andere Person in der Nähe des Benutzers befindet; und
Bestimmen, dass der Benutzer (106) während des zweiten Zeitraums nicht gehustet hat, basierend darauf, dass eine Amplitude und/oder eine Phase des weiteren akustischen Empfangssignals (504) nicht signifikant verändert ist.

7. Verfahren nach Anspruch 6, ferner umfassend:
Erfassen, unter Verwendung eines Hilfssensors (426) und während des zweiten Zeitraums, eines Over-the-Air-Sprachsignals, das durch das Husten der anderen Person erzeugt wird; und
Bestimmen, dass die andere Person während des zweiten Zeitraums gehustet hat, basierend auf dem weiteren akustischen Empfangssignal und basierend auf dem Erfassen des Over-the-Air-Sprachsignals.

8. Verfahren nach Anspruch 7, ferner umfassend:
Wahrnehmen, unter Verwendung des Hilfssensors (426) und während des ersten Zeitraums, eines weiteren Over-the-Air-Sprachsignals, das durch das Husten des Benutzers erzeugt wird, wobei das Bestimmen, dass der Benutzer (106) gehustet hat, das Bestimmen umfasst, dass der Benutzer (106) gehustet hat, basierend auf dem akustischen Empfangssignal (504) und basierend auf dem Erfassen des weiteren Over-the-Air-Sprachsignals.

9. Verfahren nach einem der vorhergehenden Ansprüche, ferner umfassend:
Senden, unter Verwendung eines weiteren Hearables (102-2), während des ersten Zeitraums, eines zweiten akustischen Sendesignals (502-2), das sich innerhalb zumindest eines Abschnitts eines zweiten Gehörgangs (114) des Benutzers (106) ausbreitet; und
Empfangen, während des ersten Zeitraums, eines zweiten akustischen Empfangssignals (504-2), wobei das zweite akustische Empfangssignal (504-2) eine Version des zweiten akustischen Sendesignals (502-2) darstellt, bei der ein oder mehrere Merkmale aufgrund der Ausbreitung innerhalb des zweiten Gehörgangs (114) und basierend darauf, dass der Benutzer (106) während zumindest eines Teils des ersten Zeitraums hustet, modifiziert sind,
wobei das Bestimmen, dass der Benutzer (106) gehustet hat, das Bestimmen umfasst, dass der Benutzer (106) während des ersten Zeitraums gehustet hat, basierend auf dem akustischen Empfangssignal (504-1) und dem zweiten akustischen Empfangssignal (504-2).

10. Nichtflüchtiges computerlesbares Speichermedium (1214), umfassend Anweisungen, die als Reaktion auf die Ausführung durch einen Prozessor (1210) ein Hearable (102; 102-1, 102-2) veranlassen, eines der Verfahren nach den Ansprüchen 1 bis 9 durchzuführen.

11. System, umfassend:
zumindest ein Hearable mit zumindest einem Wandler (406); und
zumindest einen Prozessor (414), wobei das System dazu konfiguriert ist, unter Verwendung des zumindest einen Wandlers (406) und des zumindest einen Prozessors (414) eines der Verfahren nach den Ansprüchen 1 bis 9 durchzuführen.

12. System nach Anspruch 11, ferner umfassend:
einen Lautsprecher (408); und
eine Schaltung zur aktiven Geräuschunterdrückung (424), die ein Rückkopplungsmikrofon (410) umfasst,
wobei der zumindest eine Wandler (406) den Lautsprecher (408) und das Rückkopplungsmikrofon (410) umfasst.

13. System nach Anspruch 11, umfassend ein zweites Hearable, wobei:
der zumindest eine Wandler (406) einen Lautsprecher (408) und ein Mikrofon (410) umfasst;
der Lautsprecher (408) dazu konfiguriert ist, an einem ersten Ohr (108) eines Benutzers (106) positioniert zu werden; und
das Mikrofon (410) dazu konfiguriert ist, an einem zweiten Ohr (108) des Benutzers (106) positioniert zu werden.

14. System nach einem der Ansprüche 11 bis 13, wobei das zumindest eine Hearable (402-1, 402-2) den zumindest einen Prozessor (414) umfasst.

## Revendications

1. Procédé comprenant :
la transmission (902), à l'aide d'un dispositif audible (102 ; 102-1), au cours d'une première période, d'un signal de transmission acoustique (502 ; 502-1) qui se propage dans au moins une partie d'un conduit auditif (114) d'un utilisateur (106) ;
la réception (904), à l'aide du dispositif audible (102 ; 102-1), au cours de la première période, d'un signal de réception acoustique (504 ; 504-1), le signal de réception acoustique (504 ; 504-1) représentant une version du signal de transmission acoustique (502 ; 502-1) avec une ou plusieurs caractéristiques modifiées en raison de la propagation dans le conduit auditif (114) et sur la base de la toux de l'utilisateur (106) au cours d'au moins une partie de la première période ; et
la détermination (906) que l'utilisateur (106) a toussé au cours de la première période sur la base de changements d'amplitude et/ou de phase du signal de réception acoustique (504 ; 504-1) en raison de la déformation du conduit auditif (114).

2. Procédé selon la revendication 1, comprenant également :
la génération d'un signal de commande qui commande le fonctionnement d'un dispositif sur la base de la détermination que l'utilisateur a toussé.

3. Procédé selon la revendication 2, dans lequel :
le dispositif comprend le dispositif audible (102 ; 102-1).

4. Procédé selon la revendication 2, dans lequel :
le dispositif comprend un dispositif informatique (104) qui est couplé au dispositif audible (102 ; 102-1) ;
la transmission du signal de transmission acoustique (502 ; 502-1) comprend la transmission du signal de transmission acoustique (502 ; 502-1) à l'aide du dispositif audible (102 ; 102-1) ; et la réception du signal de réception acoustique (504 ; 504-1) comprend la réception du signal de réception acoustique (504 ; 504-1) à l'aide du dispositif audible (102 ; 102-1).

5. Procédé selon la revendication 4, dans lequel la génération du signal de commande amène le dispositif informatique (104) à réaliser une surveillance de la santé sur la base de la détermination que l'utilisateur (106) a toussé.

6. Procédé selon une quelconque revendication précédente, comprenant également :
la transmission (1002), au cours d'une seconde période, d'un autre signal de transmission acoustique (502) qui se propage dans au moins une partie du conduit auditif (114) de l'utilisateur (106) ;
la réception (1004), au cours de la seconde période, d'un autre signal de réception acoustique (504), dans lequel l'utilisateur (106) ne tousse pas au cours de la seconde période et une autre personne tousse au cours d'au moins une partie de la seconde période, l'autre personne étant proche de l'utilisateur ; et
la détermination que l'utilisateur (106) n'a pas toussé au cours de la seconde période sur la base du fait qu'une amplitude et/ou une phase de l'autre signal de réception acoustique (504) n'ont pas été significativement changées.

7. Procédé selon la revendication 6, comprenant également :
la détection, à l'aide d'un capteur auxiliaire (426) et au cours de la seconde période, d'un signal vocal transmis par voie hertzienne produit par la toux de l'autre personne ; et
la détermination que l'autre personne a toussé au cours de la seconde période sur la base de l'autre signal de réception acoustique et sur la base de la détection du signal vocal transmis par voie hertzienne.

8. Procédé selon la revendication 7, comprenant également :
la détection, à l'aide du capteur auxiliaire (426) et au cours de la première période, d'un autre signal vocal transmis par voie hertzienne produit par la toux de l'utilisateur,
dans lequel la détermination que l'utilisateur (106) a toussé comprend la détermination que l'utilisateur (106) a toussé sur la base du signal de réception acoustique (504) et sur la base de la détection de l'autre signal vocal transmis par voie hertzienne.

9. Procédé selon une quelconque revendication précédente, comprenant également :
la transmission, à l'aide d'un dispositif audible supplémentaire (102-2), au cours de la première période, d'un second signal de transmission acoustique (502-2) qui se propage dans au moins une partie d'un second conduit auditif (114) de l'utilisateur (106) ; et
la réception, au cours de la première période, d'un second signal de réception acoustique (504-2), le second signal de réception acoustique (504-2) représentant une version du second signal de transmission acoustique (502-2) avec une ou plusieurs caractéristiques modifiées en raison de la propagation dans le second conduit auditif (114) et sur la base de la toux de l'utilisateur (106) au cours d'au moins une partie de la première période,
dans lequel la détermination que l'utilisateur (106) a toussé comprend la détermination que l'utilisateur (106) a toussé au cours de la première période sur la base du signal de réception acoustique (504-1) et du second signal de réception acoustique (504-2).

10. Support de stockage non transitoire lisible par ordinateur (1214) comprenant des instructions qui, en réponse à l'exécution par un processeur (1210), amènent un dispositif audible (102 ; 102-1, 102-2) à réaliser l'un quelconque des procédés selon les revendications 1 à 9.

11. Système comprenant :
au moins un dispositif audible ayant au moins un transducteur (406) ; et
au moins un processeur (414), le système étant configuré pour réaliser, à l'aide de l'au moins un transducteur (406) et de l'au moins un processeur (414), l'un quelconque des procédés selon les revendications 1 à 9.

12. Système selon la revendication 11, comprenant également :
un haut-parleur (408) ; et
un circuit d'annulation active de bruit (424) comprenant un microphone de rétroaction (410),
dans lequel l'au moins un transducteur (406) comprend le haut-parleur (408) et le microphone de rétroaction (410).

13. Système selon la revendication 11, comprenant un second dispositif audible, dans lequel :
l'au moins un transducteur (406) comprend un haut-parleur (408) et un microphone (410) ;
le haut-parleur (408) est configuré pour être positionné au niveau d'une première oreille (108) d'un utilisateur (106) ; et
le microphone (410) est configuré pour être positionné au niveau d'une seconde oreille (108) de l'utilisateur (106).

14. Système selon l'une quelconque des revendications 11 à 13, dans lequel l'au moins un dispositif audible (402-1, 402-2) comprend l'au moins un processeur (414).
